# EUROPEAN PATENT SPECIFICATION

(11) **EP 2 570 112 B1**
(45) Date of publication and mention of the grant of the patent: **21.02.2018**
(21) Application number: 11780498.9
(22) Date of filing: 25.04.2011
(51) Int. Cl.: A61K 8/37, A61K 8/31, A61Q 19/10, A61K 8/891

(54) **CLEANSING COSMETIC**
REINIGUNGSKOSMETIKUM
COSMÉTIQUE DE NETTOYAGE

(30) Priority: 14.05.2010 JP 2010112551
(43) Date of publication of application: 20.03.2013
(73) Proprietor: FUJIFILM Corporation, Tokyo 106-8620 (JP)
(72) Inventor: TASHIRO, Tomoko, Ashigarakami-gun Kanagawa 258-8577 (JP); KOSUGI, Takuji, Ashigarakami-gun Kanagawa 258-8577 (JP)
(74) Representative: Klunker IP Patentanwälte PartG mbB
(86) International application number: PCT/JP2011/060045
(87) International publication number: WO 2011/142241

(56) References cited:
- WO-A1-2009/075383
- JP-A- 2004 331 512
- JP-A- 2004 331 512
- JP-A- 2007 238 488
- JP-A- 2008 156 348
- JP-A- 2009 161 523
- DATABASE WPI Week 200917 Thomson Scientific, London, GB; AN 2009-E63559 XP002740434, & JP 2009 027958 A (NIPPON OILS&FATS CO LTD) 12 February 2009 (2009-02-12)
- DATABASE WPI Week 200801 Thomson Scientific, London, GB; AN 2008-A05759 XP002740435, & JP 2007 269749 A (FUJI FILM CO LTD) 18 October 2007 (2007-10-18)

## Description

### TECHNICAL FIELD

The present invention relates to a cleansing cosmetic.

### BACKGROUND ART

A cleansing cosmetic is a cosmetic aimed at removing oily dirt from skin utilizing a solvent effect of an oil agent. Among cleansing cosmetics, cleansing oils are known to contain a large amount of oil agents and to exhibit a favorable cleansing performance with respect to makeup cosmetics which enable long lasting makeup.

Japanese Examined Patent Application Publication No. 6-99275 discloses a non-aqueous cleanser which includes a nonionic surfactant having a predetermined HLB value and a liquid oil and is transparent or translucent, and describes that the non-aqueous cleanser has favorable feel of use such that it can be easily emulsified and dispersed in water, and can be rinsed off.

Japanese Patent Application Laid-Open (JP-A) No. 2002-284672 discloses a cleansing cosmetic including a predetermined amount of a water-soluble polyhydric alcohol, a predetermined amount of a nonionic surfactant having an HLB of from 7 to less than 12, a predetermined amount of a nonionic surfactant having an HLB of 12 or higher, and a predetermined amount of an acrylic acid-alkyl methacrylate copolymer, and also describes that the cleansing cosmetic exhibits excellent cleansing effects and provides favorable feel during and after use.

JP-A No. 2005-68082 discloses, as an oily cleanser for skin having excellent storage storability and excellent usability, an oily cleanser for skin which includes a predetermined amount of an oil component which is liquid at normal temperature, a predetermined amount of a nonionic surfactant having an HLB of from 4 to 15, and a predetermined amount of a specific divalent alcohol, but does not include water.

Various substances have been proposed as substances which may be added to such a cleansing cosmetic as an additive component, and examples of such substance include oil-soluble active substance such as a carotenoid or vitamin B and a water-soluble active substance such as vitamin C or vitamin B.

### SUMMARY OF INVENTION

### TECHNICAL PROBLEM

There may be room for further improvement in cleansing performance of a cleansing cosmetic.

In a case where an active component such as a carotenoid is added to a cleansing cosmetic as an additive component, in order to allow the additive component to exert its activity, it is preferable that the additive component uniformly dissolves in the cleansing cosmetic and be stably present in the cosmetic.

The present invention provides a cleansing cosmetic which includes a carotenoid at high solubility and high stability and has excellent cleaning performance.

### SOLUTION TO PROBLEM

Embodiments of the present invention provide the following cleansing cosmetics.
[1] A cleansing cosmetic, comprising: (A) a first oil component selected from the group consisting of tridecyl isononanoate, isopropyl palmitate, and ethylhexyl palmitate, in an amount of 30% by mass or more, (B) a liquid form emulsifier including an aliphatic hydrocarbon chain and not including an unsaturated aliphatic hydrocarbon chain, which is a polyglycerin fatty acid ester, in an amount of more than 10% by mass, and (C) a carotenoid.
[2] The cleansing cosmetic of claim 1, wherein the total amount of a second oil component having an I/O value of lower than 0.05 and a third oil component having an I/O value of higher than 0.25 is 80% by mass or less relative to the amount of the first oil component.
[3] The cleansing cosmetic of any one of claim 1 or 2, wherein the carotenoid is astaxanthin.
[4] The cleansing cosmetic of any one of [1] to [3], wherein the emulsifier comprises at least one selected from the group consisting of hexaglycerin trimyristate, hexaglycerin trilaurate, hexaglycerin tricaprylate, hexaglycerin tricaproate, decaglycerin trimyristate, decaglycerin trilaurate, decaglycerin tricaprylate, and decaglycerin tricaproate.

### DESCRIPTION OF EMBODIMENTS

Carotenoids have been known to have various physiological activities.

In a case where a carotenoid is contained in a cleansing cosmetic, the carotenoid may sometimes decompose early. Further, the solubility of carotenoid to a cleansing cosmetic is not sufficiently high. Accordingly, there have been cases in which a carotenoid does not uniformly dissolve in a cleansing cosmetic.

A cleansing cosmetic of an embodiment of the invention includes a predetermined amount of a first oil component having a medium level of I/O value, and a predetermined amount of a liquid emulsifier including an aliphatic hydrocarbon chain but not including an unsaturated hydrocarbon chain, and, thus, it can contain a carotenoid(s) at high solubility and stability, and may exhibit excellent cleansing performance.

In the present specification, a numerical range described by using the expression of "from ... to ..." represents a range including numerical values described in behind of the "from" as the minimum value and behind the "to", as the maximum value.

In the invention, in the case of referring to an amount of a component in the composition, when plural substances corresponding to the component exist in the composition, the amount means the total amount of the plural substances existing in the composition, unless noted specifically otherwise.

In the present specification an I/O value means a parameter representing a degree of hydrophilicity/hydrophobicity of a compound or a substituent. "Yuki Gainen Zu (Organic Conceptual Diagram)" (KODA, Yoshio, Sankyo Publishing Co., Ltd. 1984) describes the I/O value in detail. "I" represents inorganic property and "O" represents organic property. The higher the I/O value is, the higher the inorganic property is (which means that the polarity is high and the hydrophilicity is high).

### (A) Oil Component

The cleansing cosmetic includes a first oil component having an I/O value of from 0.05 to 0.25, in an amount of 30% by mass or more with respect to the total mass of the cleansing cosmetic.

When the amount of the first oil component in the cleansing cosmetic is 30% by mass or less, there may be a case in which the cleansing cosmetic cannot contain a carotenoid in a uniformly dissolved and stable state. From the standpoints of carotenoid solubility and carotenoid stability, the amount of the first oil component is preferably 40% by mass or more, and more preferably 50% by mass or more, with respect to the total mass of the cleansing cosmetic. From the standpoints of ensuring performance of other components which are described below, the amount of the first oil component may be 85% by mass or less, and may be 65% by mass or less, with respect to the total mass of the cleansing cosmetic.

The I/O value is preferably from 0.075 to 0.22, and more preferably 0.10 to 0.20, from the standpoints of caortenoid stability and carotenoid solubility.

The cleansing cosmetic may include other oil components which are different from the first oil component, within a range in which the effect of the invention is not hindered. Examples of other oil components include a second oil component having an I/O value of lower than 0.05 and a third oil component having an I/O value of higher than 0.25.

The second oil component is an oil component having a low polarity. From the standpoint of carotenoid solubility, the amount of the second oil component is preferably 30% by mass or less, and more preferably 20% by mass of less, with respect to the total mass of the cleansing cosmetic. The amount of the second oil component of 10% by mass or less is preferable since the cartenoid solubility is not impaired.

The third oil component is an oil component having a high polarity. From the standpoint of carotenoid decomposition, the amount of the third oil component is preferably 30% by mass or less, and more preferably 25% by mass of less, with respect to the total mass of the cleansing cosmetic. The amount of the third oil component of 20% by mass or less is preferable since the carotenoid decomposition is sufficiently suppressed, and the stability is not impaired.

The fist oil component, the second oil component and the third oil component which may be used in a cleansing cosmetic are not particularly limited as long as which are those that are usually used in a cleansing cosmetic and are liquid at 25°C. Examples thereof include ester oils, hydrocarbon oils, silicone oils, fats and oils, and mixtures thereof.

Specific examples include esters such as diethyl sebacate, cetyl 2-ethylhexanoate, isopropyl palmitate, ethylhexyl palmitate, octyldodecyl myristate, isopropyl myristate, isodecyl isononanoate, isotridecyl isononanoate, ethyl oleate and glyceryl tri(caprate/caprylate); hydrocarbon oils such as liquid paraffin, liquid isoparaffin and squalene; silicone oils such as dimethyl polysiloxane, polymethylcyclosiloxane, polyether modified methylpolycyloxan, amino modified dimethyl polysiloxane; liquid oils and fats such as olive oil, camellia oil, macadamia nut oil, castor oil, avocado oil, evening primrose oil, turtle oil, corn oil, mink oil, rapeseed oil, egg yolk oil, sesame oil, persic oil, wheat germ oil, sasanqua oil, linseed oil, cotton seed oil, perilla oil, soybean oil, peanut oil, tea seed oil, kaya oil, rice bran oil, china wood oil, tung oil, hohoba oil, germ oil, triglycerin, glycerin trioctanoate, glycerin triisopalmitate, salad oil, safflower oil, palm oil, coconut oil, peanut oil, almond oil, hazelnut oil, walnut oil, and grape seed oil.

Oil components used as the first oil component include tridecyl isononanoate (I/O = 0.14), isoprolyl palmitate (I/O = 0.16), and ethylhexyl palmitate (I/O = 0.20). Each of these may be used singly, or two or more kind thereof may be used in combination. Among these, isopropyl palmitate and ethylhexyl palmitate are preferable from the standpoint of carotenoid stability and carotenoid solubility.

When the cleansing cosmetic includes an oil component(s) other than the first oil component, the total amount of the oil components including the first oil component may be from 50% by mass to 90% by mass with respect to the total mass of the cleansing cosmetic. From the standpoint of cleansing cosmetic removal and irritating property, the total amount of the oil components is preferably from 55% by mass to 85% by mass with respect to the total mass of the cleansing cosmetic.

From the standpoints of carotenoid solubility and carotenoid stability in the cleansing cosmetic, the amount of the first oil component in the cleansing cosmetic is preferably larger than the total amount of the second oil component and the third oil component. The t amount of the second oil component and the third oil component is preferably 80% by mass or less, more preferably 60% by mass or less, and further preferably 40% by mass or less, relative to the amount of the first oil component.

### (B) Emulsifier

The cleansing cosmetic includes a liquid emulsifier including an aliphatic hydrocarbon chain and not including an unsaturated aliphatic hydrocarbon chain, in an amount of more than 10% by mass, with respect to the total mass of the cleansing cosmetic. In the invention, the state of being "liquid" means that being in a liquid form at 25°C. In a case where an emulsifier having an unsaturated aliphatic hydrocrabon chain is used, it is not possible to stably hold carotenoid(s) in the cleansing cosmetic while maintaining the emulsifier in the liquid form.

When the amount of such an emulsifier in the cosmetic composition is 10% by mass or less, the cleaning performance of the cleansing cosmetic may be impaired. From the standpoint of the cleaning performance of the cleansing composition, the amount of the emulsifier is preferably 15% by mass or more, and more preferably 20% by mass or more, relative to the total mass of the cleansing cosmetic. From the standpoints of safety and not impairing the functions of other components in the cleansing cosmetic, the amount of the emulsifier is preferably from 50% by mass or less, and more preferably 40% by mass or less, with respect to the total mass of the cleansing composition.

Polyglycerin fatty acid esters are used as liquid form emulsifiers.

The polyglycerin fatty acid ester may one kind of polyglycerin fatty acid ester or a mixture of two or more esters in which a polymerization degree of a polyglycerin is different from each other and the type of fatty acid is different from each other.

From the standpoint of HLB, at least one of the polyglycerin fatty acid ester(s) is preferably an ester of a polyglycerin having a polymerization degree of from 6 to 14 and a fatty acid having 14 or fewer carbon atoms, such as myristic acid, lauric acid, capric acid, pelargonic acid, caprylic acid, heptanoic acid or caproic acid.

Preferable examples of such a polyglycerin fatty acid ester include hexaglycerin trimyristate, hexaglycerin trilaurate, hexaglycerin tricaprylate, hexaglycerin tricaproate, decaglycerin trimyristate, decaglycerin trilaurate, decaglycerin tricaprylate and decaglycerin tricaproate.

### (C) Carotenoid

The cleansing cosmetic of the invention includes a carotenoid.

Carotenoids are colorants of terpenoids of from yellow to red, and examples thereof include naturally-occurring carotenoids such as those derived from any of plants, algae and bacteria. Carotenoids are used as naturally-occuring functional components.

On the other hand, it has been found that carotenoids function as a cleaning component since the structures of carotenoids are similar to those of lipid peroxides derived from sebum dirt. Accordingly, in the invention, a carotenoid is used as a component which may further exhibit a cleaning function.

In the present specification, the "functional component" means a component with which it can be expected that, as a result of application to a living body, a predetermined physiological effect is induced in the living body.

The carotenoids which may be used in the invention are not limited to naturally-occurring carotenoids, and any carotenoid obtained by usual methods is included in the scope of the carotenoid of the invention. For example, many of the carotenes among the carotenoids described below are also produced by synthesis, and many of commercially-available β-carotenes are synthetically produced.

Examples of the carotenoid include hydrocarbons (carotenes) and their oxidized alcohol derivatives (xanthophylls).

Examples of the carotenoid include actinioerythrol, bixin, astaxanthin, kantaxanthin, capxanthin, capsorbin, β-8'-apo-carotenal (apocarotenal), β-12'-apo-carotenal, α-carotene, β-carotene, "carotene" (a mixture of α- and β-carotenes), γ-carotene, β-cryptoxanthin, lutein, lycopene, violaxanthin, zeaxanthin and esters of a compound having a hydroxyl or carboxyl group selected from the above.

The carotenoid used in the invention is preferably oily at normal temperature from the standpoint of handling and emulsification and dispersion. Particularly preferable example is astaxanthin, which has antioxidant effects, anti-inflammatory effects, skin anti-aging effects, whitening ability and the like and which is known as yellow to red colorants. In an embodiment of the invention, the astaxanthin may be an ester formed by a fatty acid therewith.

In an embodiment, the astaxanthin may be contained in the cleansing cosmetic by using an astaxanthin-containing oil separated and extracted from a natural product as a raw material for the cleansing cosmetic. Examples of such an astaxanthin-containing oil include extracts, such as those from a culture of red yeast Phaffia, green alga Haematococcus, marine bacteria or the like, and extracts from antarctic krill and the like.

The astaxanthin may be any of the above extracted products, or a product obtained by appropriately purifying the extract as necessary, or a synthetic product. As the astaxanthins, products extracted from Haematococcus algae (hereinafter sometimes referred to as "Haematococcus alga extract") are particularly preferred from the standpoints of quality and productivity.

Specifically, Haematococcus alga extracts used in the invention may be derived from Haematococcus pluvialis, Haematococcus lacustris, Haematococcus capensis, Haematococcus droebakensis or Haematococcus zimbabwiensis.

Haematococcus alga extracts that can be used in the invention may be obtained by adding an extracting solvent such as an organic solvent such as acetone, ether, chloroform or alcohol (e.g., ethanol or methanol), or carbon dioxide in a supercritical state, followed by extraction. Commercially available products may be used.

Examples of commercially available product include ASTOTS-S, ASTOTS-2.5 O, ASTOTS-5 O and ASTOTS-10 O (trade names, manufactured by Takedashiki Co., Ltd.); AstaREAL Oil (registered trademark) 50F and AstaREAL Oil (registered trade mark) 5F manufactured by Fuji Chemical Industry Co., Ltd.; and BioAstin SCE7 (trade name, manufactured by Toyo Koso Kagaku Co., Ltd.).

In an embodiment of the invention, the amount of astaxanthin as a colorant pure component in Haematococcus alga extract is preferably from 0.001% by mass to 50% by mass, and more preferably from 0.01% by mass to 25% by mass, with respect to the total mass of the Haematococcus alga extract.

The amount of the carotenoid in the cleansng cosmetic is preferably from 0.00001% by mass to 1 % by mass, more preferably from 0.00005% by mass to 0.5 % by mass, and further preferably from 0.0001% by mass to 0.1 % by mass, with respect to the total mass of the cleansing composition.

When the amount is 0.00001% by mass or more, it tends to be favorable for exerting functional effects caused by containing the carotenoid. When the amount is 0.0001% by mass or more, high effects provided by the carotenoid can be expected. On the other hand, carotenoids are characterized to have color, and from the standpoint of color, the amount of the carotenoid is preferably 1% by mass or less, more preferably 0.5% by mass or less, and further preferably 0.1 % by mass or less, with respect to the total mass of the cleansing cosmetic.

### (D) Other Components

The cleansing cosmetic may include other components within a range in which the effect of the invention is not hindered.

The cleansing cosmetic may include vitamin E as a solvent of the carotenoid. By using vitamin E, function of vitamin E itself can be expected and the carotenoid solubility can also be improved.

When vitamin E is used as a solvent for the carotenoid, the amount of the viatmin E is preferably from 10 times to 100000 times, and more preferably from 100 times to 10000 times the mass of the carotenoid, from the standpoint of providing carotenoid solubility and carotenoid stability.

Vitamin Es are not particularly limited, and may be selected from, for example, a group of compounds consisting of tocopherol and tocopherol derivatives, and a group of compounds consisting of tocotrienol and tocotrienol derivatives. These compounds may be used singly or two or more of these compounds may be used in combination. A compound(s) selected from tocopherol or tocopherol derivatives and a compound(s) selected from tocotrienol or tocotrienol derivatives may be used in combination.

Examples of the compound selected from tocopherol or its derivatives include dl-α-tocopherol, dl-β-tocopherol, dl-γ-tocopherol, dl-δ-tocopherol; and esters of any these compounds and a carboxylic acid, for example, dl-α-tocopherol acetate, dl-α-tocopherol nicotinate, dl-α-tocopherol linoleate, and dl-α-tocopherol succinate. Among these, dl-α-tocopherol, dl-β-tocopherol, dl-γ-tocopherol, dl-δ-tocopherol and a mixutre thereof (mixed tocopherol) are preferable. Among the tocopherol derivatives, acetate esters may be preferably used.

Examples of the compound selected from tocotrienol or its derivatives include α-tocotrienol, β-tocotrienol, γ-tocotrienol, δ-tocotrienol and esters of any of these compounds and a carboxylic acid. Among tocotrienol derivatives, acetate esters may be preferably used. Tocotrienols are compounds included in, for example, wheats, rice bran or palm oil, and similar to tocopherol. Tocotrienols are compounds having three double bonds in side chains of tocopherol, and have excellent antioxidant effects.

Further, in the cleansing cosmetic of the invention, additives which are usually used in cosmetics may be used as long as the performance of the invention is not impaired. Examples thereof which may be incorporated in the cleansing composition include polyhydric alcohols, such as glycerol, 1,3-butylene glycol and propylene glycol; water-soluble polymers such as alginic acid, carboxy vinyl polymers, carboxymethyl cellulose, hydroxypropylmethyl cellulose, xanthan gum; organic or inorganic salts, such as pyrolidone carboxylic acid salts, succinic acid salts, mallic acid salts and sodium chloride; alkallis and acids as a pH adjuster; bactericides; ultraviolet absorbents; other vitamins, pigment and colorants.

### EXAMPLES

Unless otherwise indicated, "part" and "%" are based on mass.

### [Example 1]

The following components were stirred and dissolved at room temperature to obtain oil sample A. As the astaxanthin, a Haematococcus oil (trade name: ASTOTS-S, manufactured by Takedashiki Co., Ltd, astaxanthins amount: 20% by mass) was used.

| | |
|---|---|
| Ethylhexyl palmiate (I/O = 0.20) | 55 parts |
| Glyceryl tri(caprate/caprylate) (I/O = 0.30) | 20 parts |
| Polyglyceryl-10 trilaurate (HLB = 10.5) | 25 parts |
| Tocopherol | 0.5 parts |
| Haematococcus oil | 0.0052 parts |

### [Examples 2 to 3 and Comparative Examples 1 to 5]

Oil samples B to H were obtained in the same manner as in Example 1 except that the components shown in Table 1 were used.

### [Evaluation]

### 1. Carotenoid Solubility

The carotenoid solubility was evaluated in accordance with the following evaluation method.

To the oil components used in oil samples A to H, a carotenoid is added such that the amount ratio is the same as that of corresponding oil samples A to H, and the resulting mixture was stirred for 1 hour at room temperature. The evaluation of the carotenoid solubility of each oil sample was performed by visual observation, and evaluated as follows. The evaluation results are shown in Table 1.
A : Level at which the carotenoid is completely dissolved.
X: Level at which undissolved carotenoid remains.

### 2. Carotenoid Residual Ratio

With respect to oil samples A to H of each of immediately after preparation and after 1 month storage at 50°C, the carotenoid amount was measured in accordance with an ultraviolet absorbing spectrophotometry method. Based on the changes of carotenoid ultraviolet absorption, the carotenoid residual ratio in each sample was calculated. The evaluation of the residual ratio was performed as follows. The evaluation results are shown in Table 1.
A: Residual ratio of 90% or more
B: Residual ratio of 80% or more
X: Residua ratio of 80% or less

### 3. Cleaning Performance

A commercially available water-proof mascara was applied on a PET base (2cm× 2cm). 30 minutes after the application, 0.2 g of each of the oil samples was added, and mixed with the mascara so as to massage, and thereafter, the mixture was rinsed off with running water. The cleaning performance of each of the oil samples was evaluated as follows. The evaluation results are shown in Table 1.
A: The mascara was completely removed
B: The removal of the mascara was incomplete

| | Example 1 | Example 2 | Example 3 | Comparative example 1 | Comparative example 2 | Comparative example 3 | Comparative example 4 | Comparative example 5 |
|---|---|---|---|---|---|---|---|---|
| Oil Components | | | | | | | | |
| Ethylhexyl palmiate (I/O = 0.20) | 55 | 75 | 25 | 25 | 25 | 55 | 55 | 75 |
| Isopropyl palmitate (1.0 = 0.16) | | | 50 | | | | | |
| Glyceryl tri(caprate/caprylate) (I/O = 0.30) | 20 | | | 50 | | 20 | 20 | 20 |
| Mineral oil (I/O = 0) | | | | | 50 | | | |
| Emulsifier | | | | | | | | |
| Polyglyceryl-10 trilaurate | 25 | 25 | 25 | 25 | 25 | | | 10 |
| Polysorbate 80 | | | | | | 25 | | |
| Polyglyceryl-10 trioleate | | | | | | | 25 | |
| Tocopherol | 0.5 | 0.5 | 0.5 | 0.5 | 0.5 | 0.5 | 0.5 | 0.5 |
| Haematococcus oil | 0.0025 | 0.0025 | 0.0025 | 0.0025 | 0.0025 | 0.0025 | 0.0025 | 0.0025 |
| Evaluation | | | | | | | | |
| Carotenoid solubility (undissolved cartenoid) | A | A | A | A | X | A | A | A |
| Carotenoid residual ratio (%) | B | A | A | X | A | X | X | A |
| Cleaning performance | A | A | A | A | A | A | A | X |

As shown in Table 1, oil samples A to C of Examples 1 to 3, each of which includes ethylhexyl palmitate or isopropyl palmitate having a medium level of I/O value, in an amount of 30% by mass or more, and polygryceryl-10 trilaurate which does not include an unsaturated fatty acid chain, in an amount of 25% by mass or more, exhibited favorable carotenoid solubility and carotenoid stability, and also exhibited excellent cleaning performance.

According to the present invention, it is possible to provide a cleansing cosmetic which includes a carotenoid at a high stability and solubility and has excellent cleaning performance.

The disclosure of Japanese Patent Application no. 2010-112551 filed on May 14, 2010, is also referred to. All publications, patent applications, and technical standards mentioned in this specification are herein incorporated by reference to the same extent as if such individual publication, patent application, or technical standard was specifically and individually indicated are also referred to.

## Claims

1. A cleansing cosmetic, comprising:
(A) a first oil component selected from the group consisting of tridecyl isononanoate, isopropyl palmitate, and ethylhexyl palmitate, in an amount of 30% by mass or more with respect to the total mass of the cleansing cosmetic;
(B) a liquid form emulsifier including an aliphatic hydrocarbon chain and not including an unsaturated aliphatic hydrocarbon chain which is a polyglycerin fatty acid ester, in an amount of more than 10% by mass with respect to the total mass of the cleansing cosmetic; and
(C) a carotenoid.

2. The cleansing cosmetic of claim 1, wherein the total amount of a second oil component having an I/O value of lower than 0.05 and a third oil component having an I/O value of higher than 0.25 is 80% by mass or less relative to the amount of the first oil component.

3. The cleansing cosmetic of claim 1 or 2, wherein the carotenoid is astaxanthin.

4. The cleansing cosmetic of any of claims 1 to 3, wherein
the emulsifier comprises at least one selected from the group consisting of hexaglycerin trimyristate, hexaglycerin trilaurate, hexaglycerin tricaprylate, hexaglycerin tricaproate, decaglycerin trimyristate, decaglycerin trilaurate, decaglycerin tricaprylate, and decaglycerin tricaproate.

## Patentansprüche

1. Reinigungskosmetik enthaltend:
(A) eine erste Ölkomponente, ausgewählt aus der Gruppe bestehend aus Tridecylisononanoat, Isopropylpalmitat and Ethylhexylpalmitat, in einer Menge von 30 Masse% oder mehr, in Bezug auf die Gesamtmasse der Reinigungskosmetik;
(B) einen Emulgator in flüssiger Form enthaltend eine aliphatische Kohlenwasserstoffkette und nicht enthaltend eine ungesättigte aliphatische Kohlenwasserstoffkette, der ein Polyglycerylfettsäureester ist, in einer Menge von mehr als 10 Masse% in Bezug auf die Gesamtmasse der Reinigungskosmetik; und
(C) ein Carotinoid.

2. Reinigungskosmetik nach Anspruch 1, wobei die Gesamtmenge einer zweiten Ölkomponente, die einen I/O Wert von weniger als 0.05 aufweist und einer dritten Ölkomponente, die einen I/O Wert von mehr als 0.25 aufweist, 80 Masse% oder weniger in Bezug auf die Menge der ersten Ölkomponente beträgt.

3. Reinigungskosmetik nach Anspruch 1 oder 2, wobei das Carotinoid Astaxanthin ist.

4. Reinigungskosmetik nach einem der Ansprüche 1 bis 3, wobei
der Emulgator mindestens eines, ausgewählt aus der Gruppe bestehend aus Hexaglycerintrimyristat, Hexaglycerintrilaurat, Hexaglycerintricaprylat, Hexaglycerintricaproat, Decaglycerintrimyristat, Decaglycerintrilaurat, Decaglycerintricaprylat und Decaglycerintricaproat, enthält.

## Revendications

1. Cosmétique de nettoyage, comprenant :
(A) un premier composant huileux sélectionné parmi le groupe consistant en le tridécyle-isononanoate, le palmitate d'isopropyle, et le palmitate d'éthylhexyle, en une quantité supérieure ou égale à 30 % en masse ou plus par rapport à la masse totale du cosmétique de nettoyage ;
(B) un émulsifiant sous forme liquide, incluant une chaîne hydrocarbure aliphatique et n'incluant aucune chaîne hydrocarbure aliphatique non saturée qui est un ester d'acide gras de polyglycérine, en une quantité supérieure à 10 % en masse par rapport à la masse totale du cosmétique de nettoyage, et
(C) un caroténoïde.

2. Cosmétique de nettoyage selon la revendication 1, dans lequel la quantité totale d'un deuxième composant huileux présentant une valeur Inorganique/Organique (I/O) inférieure à 0,05 et d'un troisième composant huileux présentant une valeur I/O supérieure à 0,25 est inférieure ou égale à 80 % en masse par rapport à la quantité du premier composant huileux.

3. Cosmétique de nettoyage selon la revendication 1 ou 2, dans lequel le caroténoïde est de l'astaxanthine.

4. Cosmétique de nettoyage selon l'une quelconque des revendications 1 à 3, dans lequel
l'émulsifiant comprend au moins un élément sélectionné parmi le groupe consistant en le trimyristate d'hexaglycérine, le trilaurate d'hexaglycérine, le tricaprylate d'hexaglycérine, le tricaproate d'hexaglycérine, le trimyristate de décaglycérine, trilaurate de décaglycérine, le tricaprylate de décaglycérine, et le tricaproate de décaglycérine.
